# EUROPEAN PATENT APPLICATION

(11) **EP 4 841 509 A1**
(43) Date of publication of application: **18.11.2026**
(21) Application number: 25176686.1
(22) Date of filing: 15.05.2025
(51) Int. Cl.: G06N 3/047

(54) **METHOD FOR ANONYMIZING MEDICAL DATA**

(71) Applicant: Botdesign, 31400 Toulouse (FR)
(72) Inventor: Alassonniere, Stéphanie, 92340 Bourg la Reine (FR); Thuillart, Olivier, 31400 Toulouse (FR); Fontaine, Raphaël, 31300 Toulouse (FR); Fraysse, Jean Louis, 31400 Toulouse (FR)
(74) Representative: Plasseraud IP

(57) **Abstract**

The invention relates to a computer-implemented method for anonymizing medical data, the method comprising:
- providing a latent space comprising a representation of said medical data;
- learning a Riemannian metric (S2) defined on said latent space, said metric capturing a geometric structure of a distribution of said medical data within said latent space;
- an inference phase (IP) for an original medical data to be anonymized, said inference phase comprising:
-- receiving (S3) said original medical data;
-- encoding (S4) said original medical data into the latent space to obtain an original latent representation;
-- defining a synthetic latent representation by performing a local perturbation (S5) of said original latent representation, wherein said local perturbation is performed using a procedure guided by the learned Riemannian metric;
-- decoding (S6) said synthetic latent representation to generate anonymized synthetic medical data.

## Description

### Technical Field

The present document relates to the field of artificial intelligence and machine learning, specifically to data anonymization techniques utilizing generative models. More precisely, the invention concerns methods that operate on latent representations of medical data and employ Riemannian geometry to guide the generation of anonymized synthetic data. This technology is particularly applicable to medical datasets, including but not limited to continuous, categorical, and binary variables derived from electronic health records, biological measurements, or digital health devices. It is especially useful in contexts where preserving patient privacy is critical while maintaining the statistical utility of the data, such as in clinical research, multi-center studies, and data sharing under regulatory constraints. Exemplary use cases for the anonymized synthetic data include, but are not limited to, enabling open science, supporting reproducible research, facilitating the validation of machine learning models, and creating secure data sandboxes for algorithm testing and development.

### Prior art

Variational Autoencoders (VAEs) are generative artificial intelligence models widely used for creating synthetic data across various applications, notably image and data synthesis. A standard VAE typically includes an encoder neural network compressing input data into a lower-dimensional latent space and a decoder neural network reconstructing data from these latent representations. The training of standard VAEs involves optimizing these networks by minimizing reconstruction loss alongside a regularization term based on Kullback-Leibler (KL) divergence to ensure desirable latent space properties.

Conventional VAEs assume an isotropic Gaussian latent space, which does not necessarily reflect the true geometric structure or complex distributions inherent to real-world data, particularly medical data that may encompass various data modalities. Medical datasets, for example, often involve heterogeneous information, including continuous measurements (such as biomarker levels), categorical data (such as diagnostic categories), and binary variables (such as presence or absence of a symptom), each possessing distinct statistical characteristics.

Traditional data anonymization techniques often rely on perturbation, aggregation, or encryption strategies, which can significantly alter the statistical characteristics and usability of the original data. Synthetic data generation approaches such as Generative Adversarial Networks (GANs) or basic VAEs have shown promise but frequently encounter difficulties, such as mode-collapse in GANs or unrealistic data distributions due to oversimplified latent space assumptions in standard VAEs.

Recent advances have introduced geometry-aware generative models, notably Riemannian Hamiltonian Variational Autoencoders (RHVAEs), which integrate a learned Riemannian metric into the latent space. RHVAEs leverage the intrinsic geometry of data distributions, providing more faithful representations of complex and high-dimensional data. However, existing RHVAE-based approaches have primarily focused on generating realistic synthetic data rather than explicitly addressing the stringent anonymization requirements for sensitive datasets, especially medical data.

Consequently, there is a substantial need for an improved data anonymization method tailored explicitly for medical data, utilizing geometry-informed generative models.

### Summary

To this end, the present document concerns a computer-implemented method for anonymizing medical data, the method comprising:
- providing a latent space comprising a representation of said medical data;
- providing an encoder and a decoder to and from this latent space;
- learning a Riemannian metric defined on said latent space, said metric capturing a geometric structure of a distribution of said medical data within said latent space;
- an inference phase for an original medical data to be anonymized, said inference phase comprising:
   -- receiving said original medical data;
   -- encoding said original medical data into the latent space with said encoder to obtain an original latent representation;
   -- defining a synthetic latent representation by performing a local perturbation of said original latent representation, wherein said local perturbation is performed using a procedure guided by the learned Riemannian metric;
   -- decoding said synthetic latent representation using said decoder to generate anonymized synthetic medical data.

Medical data refers to any information pertaining to the health status, medical history, treatment, diagnosis, physiological measurements, biological samples, or healthcare utilization of an individual or population. This data may encompass various formats, including, but not limited to, tabular data (e.g., electronic health records, clinical trial data containing features like demographics, diagnoses, lab results, vital signs), imaging data (e.g., MRI, CT scans), signal data (e.g., ECG, EEG), genetic data, and textual data (e.g., clinical notes). In the context of the present document, this medical data is the input subject to the anonymization process.

Anonymizing medical data refers to the process of transforming original medical data into a synthetic, anonymized form. This transformation aims to significantly impede or prevent the re-identification of the individual subjects associated with the original data, while substantially preserving the statistical properties, patterns, and overall utility inherent in the original data distribution. The process involves generating new synthetic data that correspond to original data but are sufficiently altered, particularly in their latent representations, to meet anonymization criteria.

Latent space refers to a representation space, typically having a lower dimensionality than the space of the original medical data. This latent space comprises latent representations obtained by mapping or projecting the original medical data, for instance, via a trained encoder component. A key characteristic of the latent space in the context of the present document is that it possesses a learned structure, specifically a geometric structure captured by a learned Riemannian metric defined thereon, which reflects the intrinsic distribution and relationships within the original medical data. This latent space serves as an intermediate domain wherein operations such as local perturbation, guided by said learned Riemannian metric, can be performed.

Representations of medical data refers to the specific points or vectors residing within the latent space that correspond to, and are derived from, the original medical data through an encoding process. These representations capture essential features or characteristics of the original data in the lower-dimensional latent space. These representations serve as the starting points for the guided local perturbation procedure during the inference phase.

Riemannian metric refers to a mathematical structure defined on the latent space that specifies a smoothly varying inner product (dot product) on the tangent space at each point within the latent space. Functionally, it captures the local geometry of the latent space by defining how distances and angles are measured locally, reflecting the underlying structure and density of the data representations learned from the original medical data. In the context of the present document, this metric is learned from the data representations and guides the local perturbation process, ensuring that synthetic representations remain in plausible, high-density regions of the latent space.

Geometric structure of a distribution refers to the inherent shape, topology, and density variations of the medical data representations as they are arranged within the latent space. This structure often takes the form of a non-Euclidean manifold. The Riemannian metric is learned specifically to capture this intrinsic geometric structure, providing a way to understand how data relate to each other locally and identifying regions where data representations are densely clustered

Inference phase refers to the operational stage of the method where the anonymization process is applied to a specific original medical data after the encoder, decoder, the latent space structure and the Riemannian metric have already been provided or learned (e.g., during a preceding training phase for example). This phase specifically involves receiving an original data, encoding it into its original latent representation, performing the guided local perturbation using the learned metric to define a synthetic latent representation, and decoding this synthetic representation to generate the final anonymized synthetic medical data.

Encoding refers to the process of mapping an original medical data from its original data space into its corresponding latent representation within the lower-dimensional latent space. This is typically performed by an encoder component, which may be part of a larger model (like an autoencoder) trained to capture salient features of the data.

Performing a local perturbation refers to the core step of modifying an original latent representation (z_orig) to generate a synthetic latent representation (z_synth). Local perturbation signifies that z_synth is generated in the vicinity of z_orig within the latent space. A key aspect is that this perturbation is not random but is guided by the learned Riemannian metric. This guidance utilizes the geometric information encoded in the metric (e.g., local distances, density gradients) to direct the perturbation, typically through a specific procedure (such as Hamiltonian Monte Carlo adapted for the Riemannian manifold, i.e., RHMC), ensuring that z_synth is moved sufficiently far from z_orig for anonymization purposes, yet remains within a high-density data region, thus corresponding to plausible data.

Decoding refers to the process of mapping a latent representation (in this case, the synthetic latent representation z_synth) from the latent space back into the original data space, thereby generating a synthetic medical data (the anonymized synthetic medical data x_anon). This is typically performed by a decoder component, often trained conjointly with an encoder, to reconstruct data from its latent representation.

The primary advantage of the computer-implemented method defined above lies in its ability to generate high-fidelity anonymized synthetic medical data by leveraging the intrinsic geometric structure of the data distribution within a latent space. Unlike traditional anonymization techniques that often degrade data utility through excessive noise or aggregation, or standard generative models that may produce implausible data due to oversimplified assumptions about the data structure, the present invention utilizes a learned Riemannian metric to understand the "shape" of the data in the latent space. The key step of performing the local perturbation guided by this learned metric ensures that the generated synthetic latent representations, while sufficiently distanced from the original representations to achieve anonymization, are deliberately kept within high-density data regions. This targeted perturbation results in anonymized data that is not only private but also statistically realistic and plausible, preserving complex correlations and characteristics essential for meaningful downstream analysis, such as statistical modeling, machine learning model training, or data sharing for research, without significant loss of data utility compared to the original medical data. This approach is particularly beneficial for complex, heterogeneous medical datasets whose underlying structure is often non-Euclidean and poorly captured by standard methods.

Medical data may originate from various medical data acquisition sensors.

Said method may comprise a step of acquiring original medical data using said sensors, said original medical data being anonymized during the inference phase.

As used herein, the term sensors broadly refers to technical devices or components designed to detect specific physical, chemical, or biological quantities or changes in a patient's body or environment. These sensors convert measured parameters, such as pressure, temperature, electrical potential, light intensity, chemical concentration, or motion, into signals (often electrical) that can be further processed, quantified, and recorded as data, forming the basis of technical measurements.

Said sensors can be generally categorized based on the type of system they are part of. Such categories include, for example: physiological monitoring systems (prominently featuring sensors), medical imaging systems (utilizing specialized detectors, a form of sensor), laboratory analysis systems (employing various physical/chemical detection techniques), genetic and molecular analysis systems, and clinical classification and record systems (aggregating data often derived from the previous categories).

Physiological Monitoring Systems represent a primary category utilizing sensors often directly applied to or near the patient for measuring physiological and vital signs. Examples of such sensors and systems include: electrocardiogram (ECG) devices sensing electrical heart activity via electrodes; pulse oximeters sensing blood oxygen saturation using light emitters and detectors; heart-rate monitors detecting cardiac pulse; blood pressure monitors sensing arterial pressure (non-invasively via cuffs or invasively via transducers); respiratory rate sensors detecting breathing movements or airflow; thermometers measuring body temperature; and other continuous physiological monitoring systems like continuous glucose monitors (electrochemical sensors) or electroencephalogram (EEG) devices (electrodes). The signals captured by these sensors are technically processed into quantifiable numerical values (e.g., heart rate, SpO₂, blood pressure readings, temperature values) or derived features (e.g., specific ECG intervals), constituting continuous or categorical data entries.

Medical Imaging Systems technically capture anatomical or functional information. Examples include: Computed Tomography (CT) devices using X-ray detectors; Magnetic Resonance Imaging (MRI) devices detecting radiofrequency signals from nuclear spins; Positron Emission Tomography (PET) scanners detecting gamma rays from positron annihilation; X-ray radiography systems using film or digital detectors; ultrasound scanners emitting and detecting sound wave echoes; or other imaging modalities. The detectors within these systems act as sensors capturing physical phenomena. From the resulting technically acquired images, quantitative measurements are derived, such as tumor volumes, lesion sizes, intensity metrics (e.g., Hounsfield units, signal intensity values), radiomic features, or tissue property estimations, which are then structured into tabular format.

Laboratory Analysis Systems comprise laboratory analyzers and diagnostic instruments performing technical assays on biological samples. Examples include: instruments performing biochemical assays (e.g., spectrophotometers, chemical analyzers); hematology analyzers (e.g., cell counters using electrical impedance or flow cytometry); immunoassay platforms (e.g., ELISA readers using optical detection); genetic sequencing platforms; polymerase chain reaction (PCR) machines (often using fluorescence detection); or other biochemical measurement devices. These instruments provide continuous laboratory variables such as blood glucose, cholesterol levels, electrolytes, liver enzymes, or kidney function biomarkers, or categorical/binary results based on detected thresholds or presence/absence signals, all derived from technical detection processes within the instruments.

Genetic and Molecular Analysis Systems include genomic and molecular biology measurement systems used to technically analyze biological material. Examples include: DNA/RNA sequencing devices reading nucleotide sequences; microarrays measuring gene expression optically; proteomic analyzers (e.g., mass spectrometers) identifying/quantifying proteins; and similar instruments identifying the presence or absence or quantity of specific genetic or molecular markers. These technical analyses yield data such as specific gene variants (categorical/binary), expression levels (continuous), or protein quantities (continuous), forming technically derived tabular data.

The method may further comprise, for an original medical data:
- identifying its corresponding anonymized synthetic medical data generated by decoding the synthetic latent representation derived from said original medical data;
- identifying other anonymized synthetic medical data generated from other original medical data;
- measuring a first distance between said original medical data and its corresponding anonymized synthetic medical data;
- measuring one or more second distances, each second distance being between said original medical data and one of said other anonymized synthetic medical data;
- determining a measure based on a comparison between said first distance and said one or more second distances, said measure quantifying the relative proximity of said other anonymized synthetic medical data to said original medical data compared to its corresponding anonymized synthetic medical data;
wherein an anonymization criterion for said original medical data is defined based on said measure.

A distance refers to a quantitative measure of dissimilarity or difference between two data within the original medical data space. This measure can be calculated using various metrics appropriate for the nature of the medical data, including but not limited to, Euclidean distance (L2 norm), Manhattan distance (L1 norm), Hamming distance (for categorical or binary variables), cosine similarity, velastic deformation metrics (for images or shapes) or more complex domain-specific metrics designed to compare medical records or specific types of variables (continuous, categorical, binary).

The anonymization criterion, defined based on the comparison between the first distance (between an original data and its corresponding synthetic data) and the second distances (between the original data and other synthetic data), serves as a measure of anonymization effectiveness. This criterion assesses the degree to which the specific link between an original data and its synthetic counterpart is obscured within the context of the entire generated synthetic dataset. Specifically, if a significant number or percentage of other synthetic data are closer to a given original data than its own corresponding synthetic data (i.e., many second distances are smaller than the first distance), it indicates a higher level of ambiguity and makes reliable re-identification of the original data based solely on proximity to its synthetic version more difficult.

Satisfying a predefined threshold based on this measure (e.g., requiring a certain percentage of second distances to be smaller than the first distance for a majority of the original data) provides a quantifiable indication that the anonymization process has successfully reduced the uniqueness of the mapping between original and synthetic data, going beyond merely ensuring a certain perturbation magnitude or distance from the original. This approach evaluates privacy within the relational context of the generated dataset, offering a more robust check than comparisons against simple reconstruction fidelity.

The local perturbation procedure may comprise performing Riemannian Hamiltonian Monte Carlo, or RHMC, steps, the number of RHMC steps and/or a step size of the RHMC steps being adjusted based on said anonymization criterion.

Riemannian Hamiltonian Monte Carlo steps refers to the discrete iterations performed within a Hamiltonian Monte Carlo simulation, which is a Markov chain Monte Carlo (MCMC) method adapted to sample from or explore a probability distribution given in a Riemannian manifold. In the context of the present invention, these steps are specifically employed as the procedure for performing the local perturbation of an original latent representation (z_orig). Each step simulates the evolution of a system according to Hamiltonian dynamics, typically using a numerical integrator like the leapfrog algorithm, involving updates to both a position variable (corresponding to the latent representation z) and an auxiliary momentum variable (v). These dynamics, and therefore the trajectory traced by the steps, are guided by the learned Riemannian metric, which influences the potential energy (derived from the target probability density in the latent space) and kinetic energy terms of the Hamiltonian. Performing a predetermined number of these RHMC steps, potentially with a specific step size, starting from z_orig, results in the definition of the synthetic latent representation (z_synth) at the end point of the simulated trajectory.

The use of Hamiltonian Monte Carlo steps, particularly when guided by the learned Riemannian metric (i.e., Riemannian HMC), offers significant advantages for the local perturbation procedure in this anonymization method. RHMC provides an efficient mechanism for exploring the latent space locally around the original latent representation. By simulating physical dynamics, it tends to propose moves that are less correlated and more likely to be accepted (in a traditional sampling context) compared to simpler random walks, enabling a more effective yet controlled departure from the original point. In addition, the guidance provided by the learned Riemannian metric inherently incorporates the geometric structure of the data distribution into the perturbation process. The RHMC trajectory naturally follows the contours of the high-density regions defined by the metric, ensuring that the synthetic latent representation remains statistically plausible and representative of realistic data, even as it moves away from the original representation.

Furthermore, the RHMC procedure offers explicit parameters - the number of steps and the step size - that directly control the extent of the perturbation. Adjusting these parameters based on the anonymization criterion, allows for a fine-tunable and controllable degree of anonymization. Increasing the number of steps or the step size generally corresponds to a larger effective distance traveled in the latent space, providing a systematic way to balance the desired level of privacy (separation from the original data) with the need to maintain data fidelity and plausibility, as ensured by the metric guidance.

The use of RHMC steps guided a learned Riemannian metric is described in further details for example in Riemann manifold Langevin and Hamiltonian Monte Carlo methods, Mark Girolami, Ben Calderhead. JRSS-B 2011, https://doi.org/10.1111/j.1467-9868.2010.00765.x.

The number of RHMC steps and/or the step size may be adjusted such that at least a predefined percentage of a set of original data that are anonymized verifies said anonymization criterion.

For example, said predefined percentage may be selected from a range, such as between 70% and 95%, depending on the desired balance between anonymization level and data fidelity. In a preferred embodiment, the percentage is set to be at least 80%, ensuring a substantial portion of the dataset meets the anonymization criterion.

The method may further comprise, for a set of original data:
- measuring a Kullback-Leibler divergence between a distribution of the original data and a distribution of the corresponding anonymized synthetic medical data;
- verifying that said Kullback-Leibler divergence is below a predefined value, defined as a statistical fidelity criterion.

Kullback-Leibler divergence (or KL divergence) refers to a measure quantifying the difference between two probability distributions. Specifically, in this context, it measures how the probability distribution of the corresponding anonymized synthetic medical data diverges from the probability distribution of the original medical data. It is a non-negative value, where a value of zero indicates that the two distributions are identical. A low KL divergence value signifies that the overall statistical characteristics of the anonymized synthetic dataset closely resemble those of the original dataset. It serves as a measure of statistical fidelity or information loss incurred during the anonymization process.

A distribution of data (original or anonymized synthetic) refers to the way values are distributed or spread across the possible range for a given set of data (either the original medical data set or the corresponding anonymized synthetic medical data set generated by the method) within the original data space. This distribution describes the likelihood or frequency of observing different data or feature values. For complex, potentially high-dimensional medical data, this distribution can be intricate and may be represented or estimated empirically (e.g., through histograms, kernel density estimates) or parametrically for the purpose of calculating measures like the Kullback-Leibler divergence between the original and anonymized distributions.

Statistical fidelity criterion refers to the specific condition defined by the requirement that the measured Kullback-Leibler divergence is below a predefined value. This criterion serves as a global measure to ensure that the overall statistical properties of the entire set of anonymized synthetic medical data remain closely aligned with the statistical properties of the original set of medical data. Satisfying this criterion indicates that the anonymization process, while perturbing individual data for privacy, has successfully maintained the macroscopic statistical integrity and utility of the dataset as a whole. The predefined value acts as a tolerance threshold for acceptable statistical deviation.

The primary advantage of incorporating the measurement and verification of Kullback-Leibler divergence, as described above, is the provision of a global assessment of the statistical utility preserved in the anonymized dataset. While other steps might focus on the anonymization of individual data (e.g., ensuring sufficient distance from the original), this KL divergence criterion ensures that the aggregate statistical characteristics (such as marginal distributions, correlations, and potentially higher-order moments captured by the distribution) of the generated synthetic dataset do not deviate substantially from those of the original dataset.

The number of RHMC steps and/or the step size may be adjusted based on said statistical fidelity criterion.

Such adjustment may typically be implemented through a systematic, potentially iterative, procedure. This procedure may ensure that the overall statistical distribution of the generated anonymized data remains sufficiently close to that of the original data, as quantified by the Kullback-Leibler (KL) divergence falling below a predefined value.

Initially, a set of RHMC parameters (e.g., a starting number of steps N_steps and step size epsilon) may be chosen. The anonymization method, incorporating these RHMC parameters for the local perturbation, may be applied to a representative set (or the entirety) of the original medical data to generate a corresponding set of anonymized synthetic medical data. Subsequently, the KL divergence between the estimated distribution of this generated synthetic data set and the estimated distribution of the original data set may be computed.

This measured KL divergence value may then be compared to the predefined threshold value defined by the statistical fidelity criterion. If the measured KL divergence exceeds the predefined threshold, it signifies that the anonymization process, with the current RHMC parameters, has resulted in a synthetic dataset that deviates too much statistically from the original dataset. To rectify this and improve statistical fidelity, the magnitude of the perturbation may be reduced. This reduction is achieved by decreasing the number of RHMC steps (N_steps) and/or by decreasing the RHMC step size (epsilon). The process of generating the anonymized set and evaluating the KL divergence may then be repeated with these adjusted, less aggressive RHMC parameters. This iterative adjustment may continue until the measured KL divergence falls below the predefined threshold, thereby satisfying the statistical fidelity criterion.

Conversely, this adjustment mechanism allows for managing the trade-off between privacy and utility. If the primary goal is to maximize anonymization while respecting the statistical fidelity bound, one might start with minimal RHMC parameters and incrementally increase them. If the KL divergence remains below the threshold after an increase in perturbation (via increasing steps or step size), the change is accepted; otherwise, it is reverted or reduced. This ensures the statistical properties are preserved within the acceptable limit defined by the predefined KL divergence value, while potentially achieving a higher degree of anonymization as permitted by this constraint.

Said latent space representations and said Riemannian metric may be learned during a training phase using a Riemannian Hamiltonian Variational Autoencoder (RHVAE), the RHVAE comprising an encoder configured to project said medical data into the latent space and a decoder configured to reconstruct data from latent representations.

Riemannian Hamiltonian Variational Autoencoder (RHVAE) refers to a type of generative artificial intelligence model that integrates principles from Variational Autoencoders (VAEs) with concepts from Riemannian geometry and potentially Hamiltonian dynamics. Similar to a standard VAE, an RHVAE typically comprises an encoder neural network configured to project input data (such as the medical data) into a lower-dimensional latent space, thereby generating latent space representations, and a decoder neural network configured to reconstruct data from these latent representations. A key distinction of the RHVAE is its treatment of the latent space not as a standard Euclidean space, but as a Riemannian manifold. During its training phase, the RHVAE is configured to concurrently learn both the encoding/decoding functions and a Riemannian metric defined on this latent space. This learned Riemannian metric is specifically optimized to capture the intrinsic geometric structure and complex distribution patterns inherent in the input medical data.

By learning this metric directly during training, the RHVAE inherently provides both the mechanism for obtaining the latent space representations (via its trained encoder) and the learned Riemannian metric itself. This learned metric, capturing the data's intrinsic geometry, is then available for use in subsequent procedures, such as the metric-guided local perturbation step central to the claimed anonymization method performed during the inference phase. The framework may also leverage techniques derived from Reimannian Hamiltonian Monte Carlo (RHMC), adapted for the learned Riemannian geometry, within its training or sampling procedures to better navigate the structured latent space, further enhancing the quality of the learned representations and metric. Thus, employing an RHVAE as described represents one effective embodiment for obtaining the necessary structured latent space and corresponding metric required by the anonymization method.

Such RHVAE is for example known from Chadebec, C., Thibeau-Sutre, E., Burgos, N., & Allassonnière, S. (2022). Data Augmentation in High Dimensional Low Sample Size Setting Using a Geometry-Based Variational Autoencoder. IEEE Transactions on Pattern Analysis and Machine Intelligence. DOI: 10.1109/TPAMI.2022.3185773.

It should be understood that while the use of a Riemannian Hamiltonian Variational Autoencoder (RHVAE) as described above represents one effective embodiment for concurrently learning the latent space representations and the associated Riemannian metric during a training phase, the present method is not strictly limited thereto. The core anonymization method, performed during the inference phase, requires as inputs: (i) a latent space comprising representations of the medical data, accessible via an encoder, and reconstructible via a decoder, and (ii) a learned Riemannian metric defined on said latent space capturing the geometric structure of the data distribution.

Alternatively, these necessary inputs may be obtained through other means or a combination of techniques. For instance, according to another embodiment, a standard Variational Autoencoder (VAE), which assumes a simpler latent structure (e.g., isotropic Gaussian) during its initial training, can be employed first to learn the encoder, the decoder, and the initial latent space representations. Subsequently, a Riemannian metric can be learned post-hoc on this VAE-generated latent space, using the obtained latent representations. Methods for learning such a metric include, for example, deriving it from the learned covariance matrices of the VAE encoder's posterior distributions or learning a parameterized metric function fitted to the latent representations (see e.g., Chadebec, C. and Allassonnière, S., NeurIPS 2022, 'A Geometric Perspective on Variational Autoencoders' for related perspectives on VAE geometry).

In other potential embodiments, different types of autoencoders or dimensionality reduction techniques equipped with corresponding reconstruction capabilities could be used to generate the latent space and representations, provided that a suitable Riemannian metric capturing the data's geometric structure within that specific latent space can be subsequently learned or otherwise defined thereupon before performing the metric-guided local perturbation for anonymization.

The medical data may be tabular data.

Tabular medical data refers to structured healthcare datasets organized into rows representing individual patients, clinical records, or distinct medical observations, and columns corresponding to multiple patient attributes. Such data may originate from various sources, including but not limited to biological data (e.g., lab results, genetics), data collected during care provision (e.g., diagnoses, treatments, vital signs collected within a care context), prevention data, and data from digital medical devices. Each attribute, often called a variable, encodes specific types of medical, demographic, biological, genetic, or diagnostic information. Due to the diversity and complexity of medical information, these datasets frequently include heterogeneous data types such as continuous, categorical (nominal or ordinal), binary variables, and may also exhibit missing or incomplete data entries.

Continuous variables are numerical measures that exist on a continuous spectrum, typically representing biological measurements, physiological parameters, or clinical scores. They can assume any numeric value within a given interval and often follow particular statistical distributions (normal, skewed, etc.). Examples include:
- Demographic and Anthropometric Measurements: Patient age (in years), body weight (in kilograms), height (in centimeters), Body Mass Index (BMI, calculated from weight and height).
- Vital Signs and Physiological Measures: Systolic and diastolic blood pressure (mmHg), heart rate (beats per minute), respiratory rate (breaths per minute), body temperature (degrees Celsius), oxygen saturation (percentage SpO₂).
- Biochemical and Laboratory Test Results: Blood glucose concentration (mg/dL or mmol/L), cholesterol levels including high-density lipoprotein (HDL) and low-density lipoprotein (LDL), electrolytes such as sodium (Na⁺), potassium (K⁺), calcium (Ca²⁺), liver function enzymes (ALT, AST), kidney function parameters (creatinine, blood urea nitrogen).
- Clinical Scores and Indices: Severity indices such as APACHE II or SOFA scores for intensive care patients, cardiovascular risk scores (Framingham score), pain scales (visual analog scale or numeric rating scale from 0 to 10), inflammation markers (e.g., CRP levels).

Categorical variables represent qualitative information by grouping data into discrete categories. These variables can be nominal (without inherent numerical order) or ordinal (with clearly defined ordering or hierarchy).

Nominal categorical variables do not have a natural order among their categories and represent qualitative distinctions between patient attributes, diseases, or treatments. Examples include:
- Demographic Characteristics: Gender (male, female, non-binary), ethnicity or racial classification (e.g., Caucasian, African-American, Asian, Hispanic), marital status (single, married, divorced, widowed).
- Clinical and Diagnostic Information: Blood type (A, B, AB, O), diagnostic categories according to ICD-10 codes (e.g., type of diabetes, type of infection), treatment modalities (type of chemotherapy, radiotherapy, surgical procedure), pathogens or microorganisms identified (strain of influenza virus, bacterial species).
- Genetic and Molecular Information: Genotype groups (e.g., heterozygous/homozygous status for certain genetic variants), presence or absence of genetic mutations or markers (e.g., BRCA1/BRCA2 mutation).

Ordinal categorical variables contain distinct categories with a clear ranking or hierarchy among categories. They often encode severity, progression stages, or levels of clinical symptoms. Examples include:
- Disease Severity and Clinical Staging: Cancer stages (Stage I, Stage II, Stage III, Stage IV), severity classifications (mild, moderate, severe, critical), level of cognitive impairment (normal cognition, mild cognitive impairment, moderate dementia, severe dementia).
- Symptom Intensity or Pain Scales: Pain rating scales (e.g., none, mild, moderate, severe, very severe), Likert-scale ratings for patient-reported outcomes (1 = strongly disagree to 5 = strongly agree), frequency of symptoms (never, rarely, sometimes, often, always).
- Functional and Quality-of-life Measures: Mobility status (fully independent, partially dependent, fully dependent), performance status scales (e.g., ECOG performance status: grade 0 to 4, from fully active to completely disabled).

Binary variables represent attributes or clinical outcomes that have exactly two possible states, typically coded as 0/1, yes/no, true/false, or presence/absence. Binary variables simplify categorical distinctions into two clear groups. Examples in medical datasets include:
- Risk Factors and Lifestyle: Smoking status (smoker/non-smoker), alcohol consumption (regular drinker/non-drinker), physical activity (sedentary/active).
- Clinical Diagnoses and Outcomes: Presence or absence of a disease (e.g., diabetic/non-diabetic), positive or negative test results (e.g., COVID-19 PCR test positive or negative, presence or absence of specific antibodies).
- Treatment and Intervention Indicators: Whether a patient received a specific treatment or not (received/not received chemotherapy), surgery performed or not performed (yes/no), clinical response or remission achieved (responder/non-responder).
- Patient Compliance and Follow-up: Medication adherence (adherent/non-adherent), attendance to follow-up appointments (attended/missed).

The anonymized synthetic medical data generated using the present method offers considerable utility in a wide range of healthcare and biomedical research contexts. By preserving the statistical fidelity of the original dataset while ensuring that individual privacy is protected through geometry-guided perturbations in a learned latent space, the method supports the following exemplary use cases:
- Data Sharing and Open Science: The anonymized data may be safely shared across institutions, enabling collaborative research, data pooling, and open science initiatives while mitigating risks related to re-identification. This is particularly relevant in light of upcoming European regulations requiring broader data accessibility through secure "data sandboxes".
- Reproducibility and Method Validation: Researchers and developers may use the anonymized data to test hypotheses, validate computational methods, or compare algorithm performance without accessing sensitive real-world datasets. This enhances reproducibility in scientific studies and accelerates the development of new analytical tools.
- Federated and Multi-institutional Learning: Anonymized datasets can be merged across centers to simulate large, harmonized cohorts, facilitating multi-site studies and the training of robust machine learning models that require large-scale, heterogeneous data.
- Synthetic Data Sandboxes: The method enables the creation of synthetic test environments where algorithms or decision support systems can be evaluated on realistic data before deployment. These environments may be used for prototyping, regulatory testing, or educational purposes.
- Interoperability and Curation: By standardizing data format and content through a consistent anonymization pipeline, the method enhances interoperability across heterogeneous datasets and contributes to data cleaning and curation efforts.

The present document also concerns a computer program product, comprising instructions which, when executed by a processor, cause the processor to implement the above-mentioned method.

The present document also concerns a computer device comprising:
- an input interface to receive tabular medical data,
- a memory for storing at least instructions of a computer program according to the preceding claim,
- a processor accessing the memory for reading the aforesaid instructions and executing then the above-mentioned method,
- an output interface to provide generated tabular medical data.

The input interface refers to hardware and software components of the computer system that receive or acquire medical tabular data for processing. This interface may read data stored locally or remotely, in standard file formats such as plain text files (.txt), comma-separated values (.csv), Excel spreadsheets (.xlsx), or similar structured or semi-structured data files. The input interface may also support real-time data retrieval from medical databases, hospital information systems (HIS), or electronic health record (EHR) systems, ensuring compatibility with commonly used medical data formats and protocols.

The processor refers to the computational component configured to execute instructions stored in memory and carry out the training and inference phases. The processor may include one or several processing units, for instance central processing units (CPUs), graphical processing units (GPUs), or specialized artificial intelligence (AI) processing hardware such as NVIDIA Tensor Cores or Google Tensor Processing Units (TPUs). During operation, the processor executes computations necessary for optimizing neural network weights, updating the Riemannian metric, performing latent-space sampling, and generating synthetic tabular medical data according to the trained RHVAE model, for example.

The output interface refers to the component responsible for delivering the synthetic tabular medical data generated by the trained RHVAE. The output interface may provide the synthetic data in various standardized or tabular formats (e.g., csv, xlsx, txt, json), suitable for further use in clinical research, predictive modeling, or medical analyses. Additionally, the output interface may optionally integrate generated synthetic data directly into existing clinical or research databases, medical dashboards, or visualization tools, ensuring effective interpretation and immediate usability by healthcare practitioners or researchers.

### Brief description of the drawings

Other features, details and advantages will become apparent from the detailed description below, and from an analysis of the attached drawings, in which:
Figure 1 schematically illustrates a computing device according to an embodiment of the present document;
Figure 2 schematically illustrates an embodiment of the computer-implemented method for anonymizing medical data, encompassing both a training phase and an inference phase.

### Detailed description of the drawings

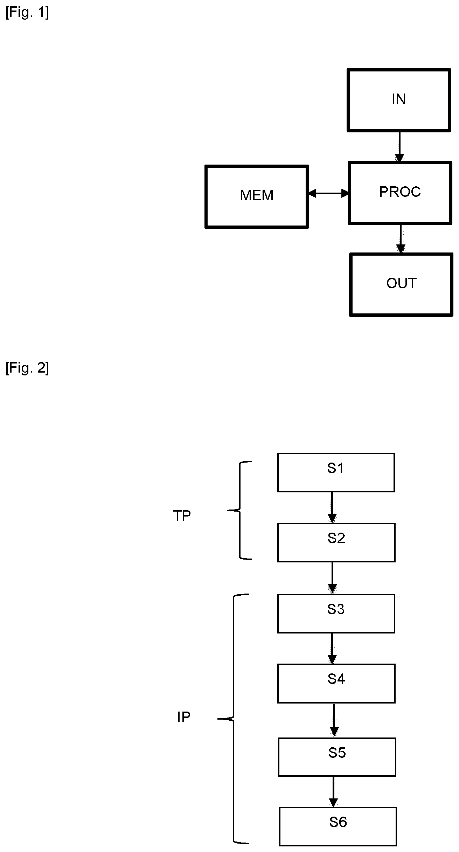

Figure 1 schematically shows an example of a computer device according to the present document.

Said computer device comprises:
- an input interface (IN) to receive medical data,
- a memory (MEM) for storing at least instructions of a computer program according to the present document,
- a processor (PROC) accessing the memory for reading the aforesaid instructions and executing then the method according to the present document,
- an output interface (OUT) to provide anonymized synthetic medical data.

Figure 2 schematically illustrates an embodiment of the computer-implemented method for anonymizing medical data, encompassing both a training phase and an inference phase.

The training phase (TP), depicted generally in the upper portion of Figure 2, involves learning the necessary components from a set of original medical data.

This phase typically comprises providing the original medical data to a generative model framework, often based on an autoencoder structure having an encoder and a decoder. During this training phase, the model parameters are optimized (S1). Specifically, the encoder is trained to map the original medical data into representations within a latent space, and the decoder is trained to reconstruct data from these latent representations. Concurrently or subsequently, a Riemannian metric is learned (S2), defined on said latent space. This metric learning process aims to capture the intrinsic geometric structure of the distribution of the medical data as represented within the latent space. The outputs of this training phase, which serve as inputs for the subsequent inference phase, are the trained encoder, the trained decoder, and the learned Riemannian metric capturing the latent space geometry.

The inference phase (IP), depicted generally in the lower portion of Figure 2, illustrates the process applied to anonymize a specific original medical data. This phase commences with receiving the original medical data (S3).

In a subsequent encoding step (S4), the received original medical data is processed by the trained encoder (obtained from the training phase). The encoder maps the original medical data into the latent space to yield its corresponding original latent representation.

Following encoding, a local perturbation step is performed (S5). This step takes the original latent representation and applies a procedure to generate a synthetic latent representation. This perturbation procedure is guided by the learned Riemannian metric (obtained from the training phase). As detailed previously, this guidance ensures the resulting synthetic latent representation is located within a region of the latent space corresponding to plausible data, while being distinct from the original latent representation. Procedures like Hamiltonian Monte Carlo (HMC) steps adapted to the Riemannian geometry may be employed here.

Finally, in a decoding step (S6), the generated synthetic latent representation is processed by the trained decoder (obtained from the training phase). The decoder maps the synthetic latent representation back from the latent space into the original data space, thereby generating the anonymized synthetic medical data, which is the output of the inference phase.

While not explicitly detailed in this schematic, it is understood that control mechanisms, potentially involving criteria such as the anonymization criterion or the statistical fidelity criterion discussed herein, may influence the parameters used within the local perturbation step during the inference phase, based on evaluations involving the original and generated anonymized data.

## Claims

1. A computer-implemented method for anonymizing medical data, the method comprising:
- providing a latent space comprising a representation of said medical data;
- providing an encoder and a decoder to and from this latent space;
- learning a Riemannian metric (S2) defined on said latent space, said metric capturing a geometric structure of a distribution of said medical data within said latent space;
- an inference phase (IP) for an original medical data to be anonymized, said inference phase comprising:
-- receiving (S3) said original medical data;
-- encoding (S4) said original medical data into the latent space with said encoder to obtain an original latent representation;
-- defining a synthetic latent representation by performing a local perturbation (S5) of said original latent representation, wherein said local perturbation is performed using a procedure guided by the learned Riemannian metric;
-- decoding (S6) said synthetic latent representation using said decoder to generate anonymized synthetic medical data.

2. The method according to the preceding claim, further comprising, for an original medical data:
- identifying its corresponding anonymized synthetic medical data generated by decoding the synthetic latent representation derived from said original medical data;
- identifying other anonymized synthetic medical data generated from other original medical data;
- measuring a first distance between said original medical data and its corresponding anonymized synthetic medical data;
- measuring one or more second distances, each second distance being between said original medical data and one of said other anonymized synthetic medical data;
- determining a measure based on a comparison between said first distance and said one or more second distances, said measure quantifying the relative proximity of said other anonymized synthetic medical data to said original medical data compared to its corresponding anonymized synthetic medical data;
wherein an anonymization criterion for said original medical data is defined based on said measure.

3. The method according to any of the preceding claims, wherein the local perturbation procedure comprises performing Riemannian Hamiltonian Monte Carlo, or RHMC, steps, and wherein the number of RHMC steps and/or a step size of the RHMC steps is adjusted based on said anonymization criterion.

4. The method according to the preceding claim, wherein the number of RHMC steps and/or the step size is adjusted such that at least a predefined percentage of a set of original data that are anonymized verifies said anonymization criterion.

5. The method according to any of the preceding claims, further comprising, for a set of original data:
- measuring a Kullback-Leibler divergence between a distribution of the original data and a distribution of the corresponding anonymized synthetic medical data;
- verifying that said Kullback-Leibler divergence is below a predefined value, defined as a statistical fidelity criterion.

6. The method according to claim 3 and claim 5, wherein the number of RHMC steps and/or the step size is adjusted based on said statistical fidelity criterion.

7. The method according to any of the preceding claims, wherein said latent space representations and said Riemannian metric are learned during a training phase (TP) using a Riemannian Hamiltonian Variational Autoencoder (RHVAE), the RHVAE comprising an encoder configured to project said medical data into the latent space and a decoder configured to reconstruct data from latent representations.

8. The method according to any of the preceding claims, wherein the medical data is tabular data.

9. A computer program product comprising instructions which, when executed by a processor, cause the processor to carry out the method according to any of the preceding claims.

10. A computing device comprising:
- an input interface (IN) configured to receive medical data;
- a memory (MEM) storing instructions of the computer program product according to the preceding claim ;
- a processor (PROC) configured to load and execute said instructions, thereby carrying out the method of any of the claims 1 to 8; and
- an output interface (OUT) for providing anonymized synthetic medical data resulting from said inference phase.
